(19) Europäisches Patentamt
European Patent Office
Office européen des brevets

(11) Veröffentlichungsnummer: **0 086 747**
**B1**

(12) # EUROPÄISCHE PATENTSCHRIFT

(45) Veröffentlichungstag der Patentschrift: **07.01.87**

(51) Int. Cl.⁴: **C 07 C 153/057**, A 01 N 37/38, A 01 N 37/52

(21) Anmeldenummer: **83810051.9**

(22) Anmeldetag: **07.02.83**

(54) **Neue Phenoxy-phenoxy-alkanoyl-thioamide als Herbizide.**

(30) Priorität: **12.02.82 CH 886/82**

(43) Veröffentlichungstag der Anmeldung: **24.08.83 Patentblatt 83/34**

(45) Bekanntmachung des Hinweises auf die Patenterteilung: **07.01.87 Patentblatt 87/2**

(84) Benannte Vertragsstaaten: **BE CH DE FR GB IT LI NL**

(56) Entgegenhaltungen:
**EP - A - 0 003 295**
**EP - A - 0 014 880**
**DE - A - 2 613 697**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel (CH)**

(72) Erfinder: **Rohr, Otto, Dr., Kilbertweg 19, CH-4106 Therwil (CH)**
Erfinder: **Pissiotas, Georg, Dr., Breslauerstrasse 8, D-7850 Lörrach (DE)**

**EP 0 086 747 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Phenoxy-phenoxy)-alkanoyl-thioamide, deren Herstellung herbizide Mittel, welche diese Thioamide als Wirkstoff enthalten, die Verwendung der Phenoxy-phenoxy-alkanoyl-thioamide als Herbizide sowie ein Verfahren zur selektiven Bekämpfung von Unkräutern, in Nutzpflanzenkulturen, vor allem in Kulturen von Getreide und Reis.

Die Phenoxy-Phenoxy-alkanoyl-thioamide entsprechen der Formel I

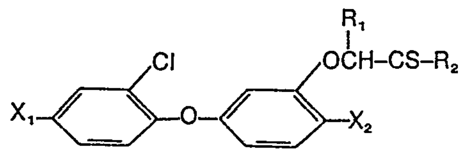
(I)

worin

$X_1$ Chlor oder Trifluormethyl,

$X_2$ Chlor oder Nitro,

$R_1$ $C_1$–$C_4$ Alkyl

$R_2$ die Amino- oder eine Amidino-Gruppe

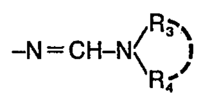

bedeutet wobei

$R_3$ und $R_4$ einzeln je Wasserstoff oder $C_1$–$C_4$ Alkyl oder zusammen eine $C_4$–$C_5$-Alkylenkette bilden.

Bevorzugte Alkylgruppen $R_1$, $R_3$ und $R_4$ sind Methyl und Äthyl.

Phenoxy-phenoxy-alkanocarbonsäurederivate mit herbizider Wirkung sind bekannt, beispielsweise aus den deutschen Offenlegungsschriften Nr. 2 223 894, 2 433 004, 2 531 643, 2 639 796 und 2 732 442 sowie den EP-A 3295 und 14 880. Sie werden zum Teil als Selektivherbizide in Getreidekulturen verwendet, wobei jedoch ihre Wirkung nicht immer befriedigt, weil zur Erreichung einer sicheren Unkrautkontrolle oft Wirkstoffmengen nötig sind, die gegenüber empfindlichen Kulturen wie Hirse oder Reis phytotoxisch wirken.

In der DE-A 2 613 697 werden Thioamide von α-(4-phenoxy-phenoxy)alkancarbonsäuren beschrieben, welche im Gegensatz zu den Thioamiden und Thioacylamidinen vorliegender Erfindung den Thioamidoalkoxyrest in para-Stellung in Bezug zur Diphenyläther-Verknüpfung haben. Sie eignen sich zur Selektiv-Bekämpfung von Schadgräsern in vorzugsweise dikolylen Kulturen auch in Getreide.

Die vorliegenden Phenoxy-phenoxy-alkanoyl-thioamide der Formel I vernichten in geringer Aufwandmenge die Unkräuter in Kulturen von Getreide und Reis oder schädigen sie so, dass sie verkümmern und keine Konkurrenz für die Kulturpflanzen mehr bedeuten.

Die Verbindungen der Formel I haben ausserdem starke pflanzenwuchsregulierende, insbesondere pflanzenwuchshemmende, Eigenschaften. Es werden sowohl Monokotyledonen als auch Dikotyledonen in ihrem Wachstum beeinträchtigt.

Besonders aktiv sind die Amide der Formel Ia

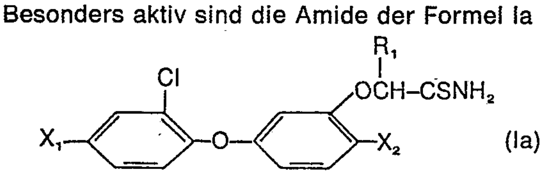
(Ia)

worin $R_1$ Methyl oder Äthyl bedeutet und $X_1$ und $X_2$ die oben angegebene Bedeutung haben, sowie die Amidine der Formel Ib,

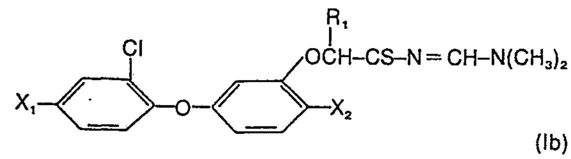
(Ib)

worin $R_1$ Methyl oder Äthyl bedeutet und $X_1$ und $X_2$ die oben angegebene Bedeutung haben.

Als Einzelverbindungen sind aufgefallen:

α-[-3-(2-Chlor-4-trifluormethylphenoxy)-6-chlorphenoxy]-thiopropionyl-amid,

α-[3-(2-Chlor-4-trifluormethylphenoxy)-6-chlorphenoxy]-thiopropionyl-dimethylamidin,

α-[3-(2,4-Dichlorphenoxy)-6-chlorphenoxy]-thiopropionyl-amid,

α-[3-(2,4-Dichlorphenoxy)-6-chlorphenoxy]-thiopropionyl-dimethylamidin,

α-[3-(2,4-Dichlorphenoxy)-6-chlorphenoxy]-thiobutyrylamid,

α-[3-(2,4-Dichlorphenoxy)-6-chlorphenoxy]-thioburyryl-dimethylamidin,

α-[3-(2,4-Dichlorphenoxy)-6-nitrophenoxy]-thiopropionyl-amid

α-[3-(2,4-Dichlorphenoxy)-6-nitrophenoxy]-thiopropionyl-dimethylamidin.

Die Phenoxy-phenoxy-alkanoyl-thioamide der Formel I sind stabile Verbindungen. Ihre Handhabung und Applikation wirft keine Probleme auf. Vorgeschlagene Aufwandmengen liegen zwischen 0,05 und 5 kg pro Hektar, vorzugsweise zwischen 0,1 und 1,5 kg.

Die Herstellung der erfindungsgemässen Phenoxy-phenoxy-alkanoyl-thioamide der Formel I erfolgt in an sich bekannter Weise durch kondensieren eines 3-(2-Chlorphenoxy)phenols der Formel II

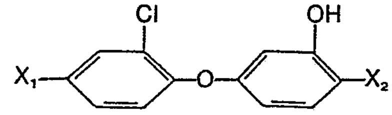
(II)

worin $X_1$ und $X_2$ die gegebene Bedeutung haben, mit einem α-Halo-$C_1$–$C_4$-alkansäurenitril der Formel III

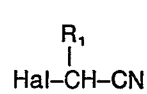
(III)

worin Hal ein Halogenatom, vorzugsweise Chlor oder Brom bedeutet und $R_1$ die gegebene Bedeutung hat und das erhaltene α-[3-(2-Chlorphenoxy)-phenoxy]-alkanoylnitril der Formel IV

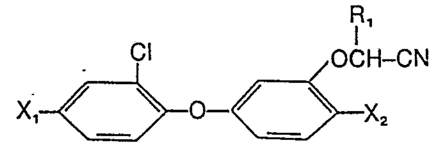
(IV)

worin $X_1$, $X_2$ und $R_1$ die gegebene Bedeutung haben, in einem inerten basischen Lösungsmittel so lange mit Schwefelwasserstoff behandelt, bis das Nitril zum Thioamid umgewandelt ist und gegebenenfalls das erhaltene Amid der Formel Ia

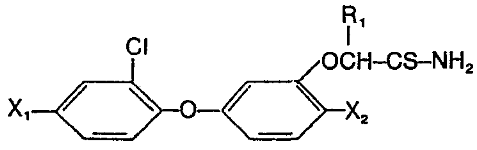 (Ia)

worin $X_1$, $X_2$ und $R_1$ die gegebene Bedeutung haben, mit einem Formamid der Formel V

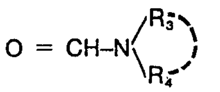 (V)

worin $R_3$ und $R_4$ die gegebene Bedeutung haben oder einem reaktionsfähigen Derivat eines solchen Formamides, in Gegenwart eines inerten organischen Lösungsmittel umsetzt.

Geeignete Derivate des Formamidins der Formel V sind beispielsweise Dimethylformamiddimethyl-acetal, Dimethylformamid-diäthylacetal, Pyrrolidino-formamid-dimethyl-acetal, Piperidinoformamid-diäthyl-acetal.

Die Reaktion wird in einem inerten organischen Lösungsmittel durchgeführt, bei Temperaturen die zwischen Raumtemperatur und 200 °C liegen. Es kann bei Normaldruck oder im Autoklaven bei Überdruck gearbeitet werden.

Geeignete basische Lösungsmittel sind z.B. Pyridin, Collidin, Dimethylformamid.

Als basische Kondensationsmittel eignen sich z.B. organische tertiäre Amine, wie das Triäthylamin oder anorganische Basen wie Natriumcarbonat-bicarbonat oder Kaliumcarbonat.

Die als Ausgangsmaterial verwendeten Phenoxy-phenole sind zum grossen Teil bekannt und ihre Herstellung ist in der neuen Patentliteratur beschrieben worden. Wir verweisen auf die DOS 2 223 894, 2 433 066, 2 433 067, 2 531 643, 2 639 796.

Die Erfindung betrifft auch herbizide und pflanzenwuchsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, insbesondere von Gräsern.

Die Verbindungen der Formel I werden in unveränderter Form oder vorzugsweise als Mittel zusammen mit den in der Formulierungstechnik üblichen Hilfsmittel eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dicotylphthalat, aliphatische Kohlenwasserstoffe wie Cyclohexan oder Paraffine, Alkohole oder Glykole sowie deren Äther und Ester, wie Äthanol, Äthylenglykol, Äthylglykolmonomethyl- oder äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$–$C_{22}$), wie z.B. die Na- oder K-Salze der Öl- oder Stearinsäure, oder von natürlichen Fettsäuregemischen, die z.B. aus Kokosnuss- oder Talgöl gewonnen werden können, genannt. Ferner sind auch die Fettsäure-methyltaurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches.

Hierher gehören auch die Salze der Schwefelsäureester und Sulfonsäuren von Fettalkohol-Äthylenoxid-Addukten. Die sulfonierten Benzimidazolderivate enthalten vorzugsweise 2-Sulfonsäuregruppen und einen Fettsäurerest mit 8–22 C-Atomen. Alkylarylsulfonate sind z. B. die Na-, Ca- oder Triäthanolaminsalze der Dodecylbenzolsulfonsäure, der Di-butylnaphthalinsulfonsäure oder eines Naphthalinsulfonsäure-Formaldehydkondensationsproduktes.

Ferner kommen auch entsprechende Phosphate wie z. B. Salze des Phosphorsäureesters eines p-Nonylphenol-(4–14)-Äthylenoxid-Adduktes in Frage.

Als ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykoläthergruppen und 10 bis 100 Propylenglykoläthergruppen enthaltenden Polyäthylenoxidaddukte an Polypropylenglykol mit 1 bis 10 Kohlenstoffatomen in der Alkylkette. Die genannten Verbindungen enthalten üblicherweise pro Propylenglykol-Einheit 1 bis 5 Äthylenglykoleinheiten.

Als Beispiele nichtionischer Tenside seien Nonylphenolpolyäthoxy-äthanole, Ricinusölpolyglykoläther, Polypropylen-Polyäthylenoxidaddukte, Tributylphenoxypolyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl oder niedrige Hydroxyalkylreste aufweisen. Die Salze liegen vorzugsweise als Halogenide, Methylsulfate oder Äthylsulfate vor, z. B. das Stearyltrimethylammoniumchlorid oder das Benzyldi(2-chloräthyl)-äthylammoniumbromid.

Die in der Formulierungstechnik gebräuchlichen Tenside sind u. a. in folgenden Publikationen beschrieben:

«Mc Cutcheon's Detergents and Emulsifiers Annual» MC
Publishing Corp., Ringwood, New Jersey, 1979.
Sisely and Wood, «Encyclopedia of Surface Active Agents».
Chemical Publishing Co., Inc. New York, 1964.

Die pestiziden Zubereitungen enthalten in der Regel 0,1 bis 95%, insbesondere 0,1 bis 80%, Wirkstoff der Formel I, 1 bis 99,9% eines festen oder flüssigen Zusatzstoffes und 0 bis 25%, insbesondere 0,1 bis 25% eines Tensides.

Insbesondere setzen sich bevorzugte Formulierungen folgendermassen zusammen: (% = Gewichtsprozent)

| | |
|---|---|
| **Emulgierbare Konzentrate** | |
| Aktiver Wirkstoff: | 1 bis 20%, bevorzugt 5 bis 10% |
| oberflächenaktives Mittel: | 5 bis 30%, vorzugsweise 10 bis 20% |
| flüssiges Trägermittel: | 50 bis 94%, vorzugsweise 70 bis 85%. |
| **Stäube** | |
| Aktiver Wirkstoff: | 0,1 bis 10%, vorzugsweise 0,1 bis 1% |
| festes Trägermittel: | 99,9 bis 90%, vorzugsweise 99,9 bis 99%. |
| **Suspension-Konzentrate** | |
| Aktiver Wirkstoff: | 5 bis 75%, vorzugsweise 10 bis 50% |
| Wasser: | 94 bis 25%, vorzugsweise 90 bis 30% |
| oberflächenaktives Mittel: | 1 bis 40%, vorzugsweise 2 bis 30%. |
| **Benetzbare Pulver** | |
| Aktiver Wirkstoff: | 0,5 bis 90%, vorzugsweise 1 bis 80% |
| oberflächenaktives Mittel: | 0,5 bis 20%, vorzugsweise 1 bis 15% |
| festes Trägermaterial | 5 bis 95%, vorzugsweise 15 bis 90% |
| **Granulate** | |
| Aktiver Wirkstoff: | 0,5 bis 30%, vorzugsweise 3 bis 15% |
| festes Trägermittel: | 99,5 bis 70%, vorzugsweise 97 bis 85%. |

Während als Handelsware eher konzentrierte Mittel bevorzugt werden, verwendet der Endverbraucher in der Regel verdünnte Mittel. Die Anwendungsformen können bis hinab zu 0,001% an Wirkstoff verdünnt werden. Die Aufwandmengen betragen in der Regel 0,01 bis 10 kg AS/ha, vorzugsweise 0,025 bis 5 kg AS/ha.

Die Mittel können auch weitere Zusätze wie Stabilisatoren, Entschäumer, Viskositätsregulatoren, Bindemittel, Haftmittel sowie Dünger oder

andere Wirkstoffe zur Erzielung spezieller Effekte enthalten.

In den folgenden Beispielen sind die Temperaturen in Celsiusgraden °C, die Drucke in Millibar mb angegeben.

Beispiel 1:
Herstellung von
α-[3-(2'-Chlor-4'-trifluormethylphenoxy)-6-chlorphenoxy]thiopropionyl-amid

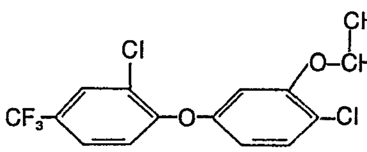

Verbindung Nr. 1

a) Man rührt über Nacht bei 100° Badtemperatur eine Mischung von 60 g
3-Hydroxy-2',4-dichlor-4'-trifluormethyl-diphenyläther,
42,9 g Brompropionitril, 51,5 g Kaliumcarbonat, und eine Spatelspitze Kaliumjodid. Nach dem Abkühlen wird das Reaktionsgemisch filtriert und das Filtrat eingedampft. Es bleiben 55 g
α-[3-(2'-Chlor-4'-trifluormethylphenoxy)-6-chlorphenoxy]propionitril
als rotbraunes Öl vom Brechungsindex $n_D^{24}$ 1.5291 zurück.

b) Man läst 37,6 g dieses Propionitrils in 35 ml Pyridin, gibt 14 ml Triäthylamin dazu und leitet dann unter Rühren während 2 Stunden Schwefelwasserstoff in die Lösung. Nach beendeter Reaktion wird die Lösung in Wasser gegossen und die organische Phase mit Äthylacetat extrahiert. Nach Trocknen über Natriumsulfat, Verdampfen des Lösungsmittels und Reinigung des Rückstandes durch Chromatographie über eine Silikagelkolonne, erhält man das Titelprodukt als rotes Öl, welches beim Stehen kristallisiert. Ausbeute: 37 g kristallines Produkt mit Schmelzpunkt 83–89 °C.

Beispiel 2:
Herstellung von
α-[3-(2'-Chlor-4'-trifluormethylphenoxy)-6-chlorphenoxy]thiopropionyl-dimethylamidin

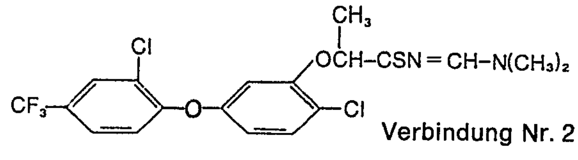

Verbindung Nr. 2

Man rührt während 3 Stunden bei 40 °C 20,5 g des gemäss Beispiel 1 erhaltenen
α-[3-(2'-Chlor-4'-trifluormethylphenoxy)-6-chlorphenoxy]-thiopropionyl-amides
zusammen mit 25 ml
N,N-Dimethylformamid-dimethylacetal
$[(CH_3)_2-N-CH(OCH_3)_2]$.
Nach dem Abkühlen wird das überschüssige Acetal abdestilliert und es bleiben 20,6 g Titelprodukt als viskoses Öl zurück.

In analoger Weise zu diesen Beispielen erhält man folgende Verbindungen

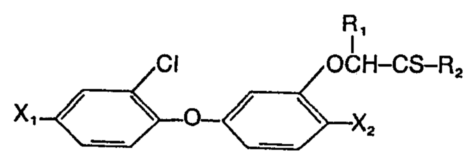

| Verbindung Nr. | $X_1$ | $X_2$ | $R_1$ | $R_2$ | phys. Daten |
|---|---|---|---|---|---|
| 1 | $CF_3$ | Cl | $CH_2$ | $NH_2$ | Smp. 83–89° |
| 2 | $CF_3$ | Cl | $CH_3$ | $N=CH-N(CH_3)_2$ | viskoses Öl |
| 3 | Cl | Cl | $CH_3$ | $NH_2$ | Smp. 93–95° |
| 4 | Cl | Cl | $CH_3$ | $N=CH-N(CH_3)_2$ | viskoses Öl |
| 5 | Cl | Cl | $C_2H_5$ | $NH_2$ | $n_D^{26}$ 1,6660 |
| 6 | Cl | Cl | $C_2H_5$ | $N=CH-N(CH_3)_2$ | viskoses Öl |
| 7 | Cl | $NO_2$ | $CH_3$ | $NH_2$ | viskoses Öl |
| 8 | Cl | $NO_2$ | $CH_3$ | $N=CH-N(CH_3)_2$ | viskoses Öl |
| 9 | $CF_3$ | $NO_2$ | $CH_3$ | $NH_2$ | viskoses Öl |
| 10 | $CF_3$ | $No_2$ | $CH_3$ | $N=CH-N(CH_3)_2$ | viskoses Öl |

Beispiel 3:
Formulierungsbeispiele für Wirkstoffe der Formel I (% = Gewichtsprozent)

| a) Spritzpulver | a) | b) | c) |
|---|---|---|---|
| α-[3-(2',4'-Dichlorphenoxy)-6-chlorphenoxy]-thiopropionyl-amid | 20% | 60% | 0,5% |
| Na-Ligninsulfonat | 5% | 5% | 5 % |
| Na-Laurylsulfat | 3% | – | – |
| Na-Diisobutylnaphthalinsulfonat | – | 6% | 6 % |
| Octylphenolpolyäthylenglykoläther (7–8 Mol AeO | – | 2% | 2 % |
| Hochdisperse Kieselsäure | 5% | 27% | 27 % |
| Kaolin | 67% | – | – |
| Natriumchlorid | – | – | 59,5% |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

b) Emulsion-Konzentrat

| | a) | b) |
|---|---|---|
| α-[3-(2′,4′-Dichlor-phenoxy)-6-chlorphen-oxy]-thiobutyryl-amid | 10% | 1% |
| Octylphenolpolyäthylenglykoläther (4–5 Mol AeO) | 3% | 3% |
| Ca-Dedecylbenzolsulfonat | 3% | 3% |
| Rincinusölpolyglykoläther (36 Mol Aeo) | 4% | 4% |
| Cyclohexanon | 30% | 10% |
| Xylolgemisch | 50% | 79% |

Aus diesem Konzentrat können durch Verdünnen mit Wasser Emulsionen jeder gewünschten Konzentration hergestellt werden.

c) Stäubemittel

| | a) | b) |
|---|---|---|
| α-[3-(2′-Chlor-4′-trifluormethyl-phenoxy)-6-chlorphenoxy]thio-propionylamid | 0,1% | 1% |
| Talkum | 99,9% | – |
| Kaolin | – | 99% |

Man erhält anwendungsfertige Stäubemittel, indem der Wirkstoff mit dem Träger vermischt und auf einer geeigneten Mühle vermahlen wird.

d) Extruder Granulat

| | a) | b) |
|---|---|---|
| α-[3-(2′,4′-Dichlorphenoxy)-6-nitrophenoxy]-thiopropionyl-amid | 10% | 1% |
| Na-Ligninsulfonat | 2% | 2% |
| Carboxymethylcellulose | 1% | 1% |
| Kaolin | 87% | 96% |

Der Wirkstoff wird mit den Zusatzstoffen vermischt, vermahlen und mit Wasser angefeuchtet. Dieses Gemisch wird extrudiert und anschliessend im Luftstrom getrocknet.

e) Umhüllungs-Granulat

| | |
|---|---|
| α-[3-(2′,4′-Dichlorphenoxy)-6-chlorphenoxy]-thiopropionyl-dimethylamidin | 3% |
| Polyäthylenglykol (MG 200) | 3% |
| Kaolin | 94% |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

f) Suspensions-Konzentrat

| | a) | b) |
|---|---|---|
| α-[3-(2′,4′-Dichlorphenoxy)-6-chlorphenoxy)-thiobutyryl-dimethylamidin | 40 % | 5 % |
| Äthylenglykol | 10 % | 10 % |
| Nonylphenolpolyäthylenglykoläther (15 Mol AwO) | 6 % | 1 % |
| Na-Ligninsulfonat | 10 % | 5 % |
| Carboxymethylcellulose | 1 % | 1 % |
| 37%ige wässrige Formaldehyd-Lösung | 0,2% | 0,2% |
| Silikonöl in Form einer 75%igen wässrigen Emulsion | 0,8% | 0,8% |
| Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

g) Salzlösung

| | |
|---|---|
| α-[3-(2′-Chlor-4′-trifluormethyl-phenoxy)-6-chlrophenoxy]-thiopropionyl-dimethylamidin | 5% |
| Isopropylamin | 1% |
| Octylphenolpolyäthylenglykoläther (78 Mol AeO) | 3% |
| Wasser | 91% |

Biologische Beispiele

Beispiel 4: Nachweis der Herbizidwirkung

Herbizide Wirkung bei Applikation der Wirkstoffe nach dem Auflaufen der Pflanzen (post-emergent).

Verschiedene Kulturpflanzen und Unkräuter werden aus den Samen in Töpfen im Gewächshaus aufgezogen, bis sie das 4- bis 6-Blatt-Stadium erreicht haben. Dann werden die Pflanzen mit wässerigen Wirkstoffemulsionen in verschiedenen Konzentrationen gespritzt. Die behandelten Pflanzen werden dann bei optimalen Bedingungen von Licht, Begiessung, Temperatur (22–25 °C) und 50–70% relativer Luftfeuchtigkeit gehalten. Nach 2 Wochen wird der Versuch ausgewertet und der Zustand der Pflanzen gemäss folgender Skala beurteilt.

- 9 = Pflanze gedeiht wie unbehandelte Kontrolle keine phytotoxischen Symptome
- 7–8 = leichte reversible Schäden
- 6 = reversibler Schaden
- 5 = 50% Schaden
- 4 = irreversibler Schaden
- 2, 3 = schwere Schäden, Pflanze verkümmert
- 1 = Pflanze abgestorben

Die Resultate sind untenstehend zusammengefasst.

In einem weiteren Versuch wird die Eignung der Verbindung Nr. 1, Unkräuter selektiv in Reiskulturen zu bekämpfen untersucht. Als Vergleichssubstanzen dienen verschiedene bekannte Herbizide

A) 4-Nitro-2′,4′,6′-trichlor-diphenyl-äther
bekannt aus dem US Patent No. 3 080 225

B) 2′,4′-Dichlor-4-nitro-diphenyl-äther «nitrofen»
bekannt aus dem US Patent Nr. 3 080 225

C) 2-Chlor-2′,6′-diäthyl-N-(butoxymethyl)-acetanilid «butachlor»,
bekannt aus dem US Patent Nr. 3 442 945

D) O-[β-naphthyl]-milchsäureanilid
bekannt aus dem US Patent Nr. 3 489 671

E) 1,3-Dimethyl-4-(3′,4′-dichlorbenzoyl)-5-para-tolylsulfonyl-imidazol,
bekannt aus DOS 2 513 750.

Der Versuch wird wie folgt durchgeführt:

Reispflänzchen, welche 25 Tage alt sind, werden im Gewächshaus in grosse rechteckige Eternitschalen verpflanzt. Zwischen die Reihen der

| Verbindung No. | 1 | | | 2 | | | 4 | | | 5 | | | 6 | | |
|---|---|---|---|---|---|---|---|---|---|---|---|---|---|---|---|
| Aufwandmenge kg/ha | 1/2 | 1/4 | 1/8 | 1 | 1/2 | 1/4 | 2 | 1 | 1/2 | 2 | 1 | 1/2 | 2 | 1 | 1/2 |
| Pflanze | | | | | | | | | | | | | | | |
| Weizen | 4 | 6 | 7 | 6 | 7 | 7 | 7 | 9 | 9 | 6 | 8 | 9 | 6 | 7 | 9 |
| Mais | 3 | 3 | 5 | 5 | 5 | 6 | 6 | 7 | 8 | 8 | 8 | 9 | 6 | 7 | 8 |
| Reis | 5 | 7 | 9 | 4 | 5 | 7 | 9 | 9 | 9 | 7 | 7 | 9 | 7 | 7 | 9 |
| Abutilon sp. | 1 | 1 | 1 | 1 | 2 | 2 | 2 | 4 | 6 | 2 | 2 | 3 | 2 | 3 | 5 |
| Xanthium sp. | 1 | 1 | 2 | 1 | 1 | 3 | 3 | 4 | 5 | 2 | 2 | 3 | 2 | 3 | 5 |
| Chenopodium album | 1 | 1 | 1 | 1 | 1 | 2 | 2 | 2 | 2 | 2 | 2 | 2 | 2 | 2 | 3 |
| Ipomoea purpurea | 1 | 1 | 1 | 1 | 2 | 4 | 2 | 2 | 3 | 2 | 2 | 3 | 1 | 1 | 3 |
| Sinapis alba | 1 | 1 | 1 | 1 | 1 | 1 | 2 | 2 | 2 | 2 | 2 | 3 | 3 | 4 | 4 |
| Galium aparine | 1 | 1 | 1 | 2 | 2 | 2 | 2 | 3 | 4 | 1 | 1 | 3 | 3 | 4 | 5 |
| Viola tricolor | 1 | 1 | 1 | 1 | 1 | 1 | 2 | 3 | 4 | 1 | 1 | 1 | 1 | 2 | 2 |

Reispflanzen werden dann Samen der in Reiskulturen vorkommenden Unkräuter Echinochloa crus galli, Gyperus difformis, Ammania und Rotala gesät. Die Schalen werden gut bewässert und bei einer Temperatur von ca. 25 °C und hoher Luftfeuchtigkeit gehalten. Nach 12 Tagen, wenn die Unkräuter aufgelaufen sind und das 2–3 Blattstadium erreicht haben, wird die Erde in der Schale mit einer 2,5 cm hohen Schicht Wasser bedeckt. Die Verbindungen werden dann als Emulsionskonzentrate mittels einer Pipette zwischen die Pflanzenreihe appliziert, wobei man das Emulsionskonzentrat so verdünnt und aufträgt, dass es einer Applikationsmenge im Feld von 2 kg Wirkstoff pro Hektar entspricht. Der Versuch wird nach 4 Wochen ausgewertet. Die Bewertung der Pflanzen geschieht gemäss der oben gegebenen Skala. Die Resultate sind in der folgenden Tabelle zusammengefasst.

| Verbindung | Nr. 1 | A | B | C | D | E |
|---|---|---|---|---|---|---|
| Pflanze | | | | | | |
| Reis | 7 | 9 | 2 | 3 | 9 | 8 |
| echinochloa crus galli | 1 | 9 | 1 | 1 | 9 | 7 |
| cyperus rotundus | 1 | 5 | 1 | 8 | 9 | 5 |
| ammania indica | 1 | 9 | – | 8 | 1 | 6 |
| rotala indica | 2 | 9 | 2 | 8 | 9 | 9 |

Die Verbindung Nr. 1 vernichtet oder schädigt die Unkräuter sehr stark während der Reis nur ganz unbedeutende Schäden aufweist.

**Patentansprüche**

1. Neue Phenoxy-phenoxy-alkanoyl-thioamide der Formel I

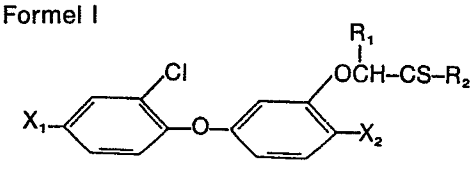

(I)

worin

$X_1$ Chlor oder Trifluormethyl,

$X_2$ Chlor oder Nitro,

$R_1$ $C_1$–$C_4$ Alkyl

$R_2$ die Aminogruppe oder ein Amidinorest

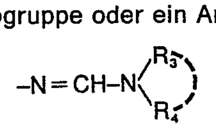

worin

$R_3$ und $R_4$ je einzeln Wasserstoff oder $C_1$–$C_4$ Alkyl und zusammen $C_4$–$C_5$ Alkylen bedeuten.

2. Amide gemäss Anspruch 1 der Formel Ia

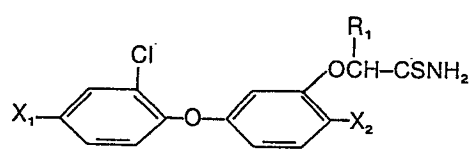

(Ia)

worin $X_1$, $X_2$ und $R_1$ die im Anspruch 1 gegebene Bedeutung haben.

3. Amidine gemäss Anspruch 1 der Formel Ib

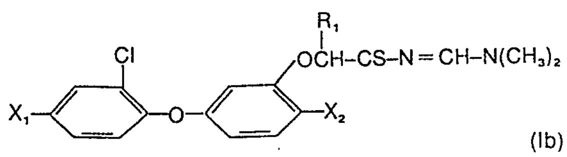

(Ib)

worin $X_1$, $X_2$ und $R_1$ die im Anspruch 1 gegebene Bedeutung haben.

4. α-[3-(2′-Chlor-4′-trifluormethylphenoxy)-6-chlorphenoxy]-thiopropionyl-amid gemäss Anspruch 1.

5. α-[3-(2′,4′-Dichlorphenoxy)-6-chlorphenoxy]-thiopropionyl-amid gemäss Anspruch 1.

6. α-[3-(2′,4′-Dichlorphenoxy)-6-nitrophenoxy]-thiopropionyl-amid gemäss Anspruch 1.

7. α-[3-(2′,4′-Dichlorphenoxy)-6-chlorphenoxy]-thiobutyryl-amid gemäss Anspruch 1.

8. α-[3-(2′-Chlor-4′-trifluormethylphenoxy)-6-chlorphenoxy]-thiopropionyl-dimethylamidin gemäss Anspruch 1.

9. α-[3-(2′,4′-Dichlorphenoxy)-6-chlorphenoxy]-thiopropionyl-dimethylamidin, gemäss Anspruch 1.

10. α-[3-(2′,4′-Dichlorphenoxy)-6-nitrophenoxy]-thiopropionyl-dimethylamidin, gemäss Anspruch 1.

11. α-[3-(2′,4′-Dichlorphenoxy)-6-chlorphenoxy]-thiobutyryl-dimethylamidin gemäss Anspruch 1.

12. Verfahren zur Herstellung von neuen Phenoxy-phenoxy-alkanoyl-thioamiden der Formel I, Anspruch 1, dadurch gekennzeichnet, dass man 3-(2-Chlorphenoxy)-phenol der Formel II

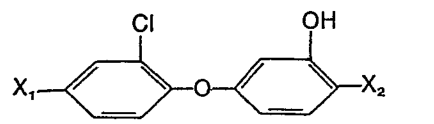 (II)

worin $X_1$ und $X_2$ die im Anspruch 1 gegebene Bedeutung haben, mit einem α-Halo-$C_1$–$C_4$-alkancarbonsäurenitril der Formel III kondensiert,

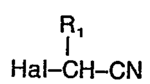 (III)

worin Hal ein Halogenatom bedeutet und $R_1$ die im Anspruch 1 gegebene Bedeutung hat und das erhaltene α-[3-(2-Chlorphenoxy)-phenoxy-alkanoylnitril der Formel IV

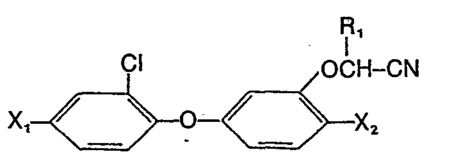 (IV)

worin $X_1$, $X_2$ und $R_1$ die im Anspruch 1 gegebene Bedeutung haben, in einem inerten basischen Lösungsmittel mit Schwefelwasserstoff so lange behandelt bis das Nitril zum Thiamid umgewandelt ist und gegebenenfalls das erhaltene Amid der Formel Ia

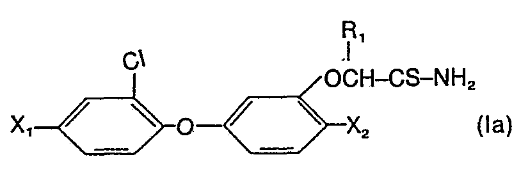 (Ia)

worin $X_1$, $X_2$ und $R_1$ die im Anspruch 1 gegebene Bedeutung haben, weiter mit einem Formamid der Formel V

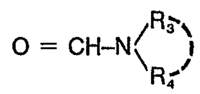 (V)

worin $R_3$ und $R_4$ die im Anspruch 1 gegebene Bedeutung haben, oder mit einem reaktionsfähigen Derivat eines solchen Formamides, in Gegenwart eines inerten organischen Lösungsmittels und eines basischen Kondensationsmittels umsetzt.

13. Herbizides Mittel, dadurch gekennzeichnet, dass es neben inerten Träger- und Verteilermaterial als aktive Komponente ein Phenoxy-phenoxy-alkanoyl-thioamid der Formel I erhält.

14. Verfahren zum selektiven Bekämpfen von Unkräutern in Nutzpflanzenkulturen, dadurch gekennzeichnet, dass man die Kulturen oder deren Anbaufläche mit einer wirksamen Menge eines Phenoxy-phenoxy-alkanoyl-thioamides gemäss Anspruch 1 handelt.

15. Verfahren gemäss Anspruch 14 zum Bekämpfen von Unkräutern in Getreide und Reiskulturen.

16. Die Verwendung der Phenoxy-phenoxyalkoxy-thioamide gemäss Anspruch 1 zur Unkrautbekämpfung in Kulturen von Getreide und Reis.

## Claims

1. A novel phenoxyphenoxyalkanoylthioamide of the formula I

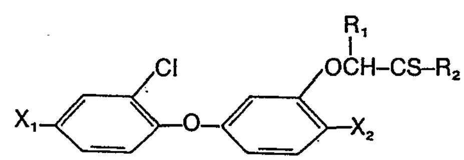 (I)

wherein $X_1$ is chlorine or trifluoromethyl, $X_2$ is chlorine or nitro, $R_1$ is $C_1$–$C_4$alkyl, and $R_2$ is the amino group or an amidino group

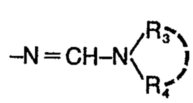

wherein each of $R_3$ and $R_4$ independently is hydrogen or $C_1$–$C_4$alkyl or taken together they form a $C_4$–$C_5$alkylene chain.

2. An amide according to claim 1 of the formula Ia

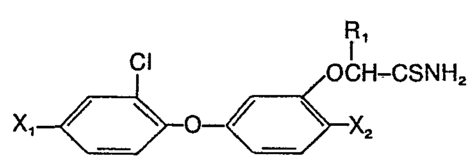 (Ia)

wherein $X_1$, $X_2$ and $R_1$ are as defined in claim 1.

3. An amidine according to claim 1 of the formula Ib

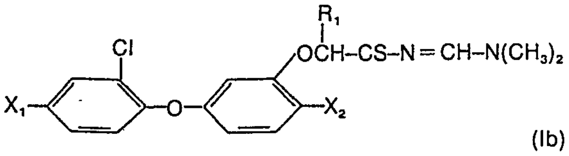

(Ib)

wherein $X_1$, $X_2$ and $R_1$ are as defined in claim 1.

4. α-[3-(2′-Chloro-4′-trifluoromethylphenoxy-6-chlorophenoxy]thiopropionylamide according to claim 1.

5. α-[3-(2′,4′-Dichlorophenoxy)-6-chlorophenoxy]-thiopropionylamide according to claim 1.

6. α-[3-(2′,4′-Dichlorophenoxy)-6-nitrophenoxy]-thiopropionylamide according to claim 1.

7. α-[3-(2′,4′-Dichlorophenoxy)-6-chlorophenoxy]-thiobutyrylamide according to claim 1.

8. α-[3-(2′-Chloro-4′-trifluoromethylphenoxy)-6-chlorophenoxy]thiopropionyl-dimethylamidine according to claim 1.

9. α-[3-(2′,4′-Dichlorophenoxy)-6-chlorophenoxy]-thiopropionyl-dimethylamidine according to claim 1.

10. α-[3-(2′,4′-Dichlorophenoxy)-6-nitrophenoxy]-thiopropionyl-dimethylamidine according to claim 1.

11. α-[3-(2′,4′-Dichlorophenoxy)-6-chlorophenoxy]-thiobutyryl-dimethylamidine according to claim 1.

12. A process for the preparation of a novel phenoxyphenoxyalkanoylthioamide of the formula I according to claim 1, which process comprises condensing 3-(2-chlorophenoxy)-phenol of the formula II

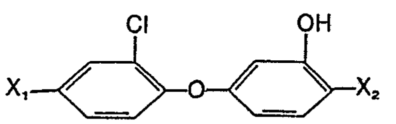

(II)

wherein $X_1$ and $X_2$ are as defined in claim 1, with an α-halo($C_1$–$C_4$)alkanecarboxylic acid nitrile of the formula III

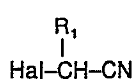

(III)

wherein Hal is a halogen atom and $R_1$ is as defined in claim 1, and treating the resultant α-[3-(2-chlorophenoxy)-phenoxy]alkanoylnitrile of the formula IV

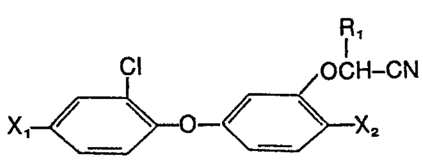

(IV)

wherein $X_1$, $X_2$ and $R_1$ are as defined in claim 1, in an inert basic solvent, with hydrogen sulfide until the nitrile is converted to the thioamide and, if desired, reacting the amide of the formula Ia

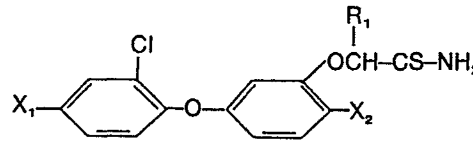

(Ia)

wherein $X_1$, $X_2$ and $R_1$ are as defined in claim 1, with a formamide of the formula V

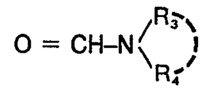

(V)

wherein $R_3$ and $R_4$ are as defined in claim 1, or with a reactive derivative thereof, in the presence of an inert organic solvent and of a basic condensing agent.

13. A herbicidal composition which contains a phenoxyphenoxyalkanoylthioamide of the formula I as active ingredient, together with inert carriers and adjuvants.

14. A method of selectively controlling weeds in crops of useful plants, which comprises treating said crops or the locus thereof with a herbicidally effective amount of a phenoxyphenoxyalkanoylthioamide according to claim 1.

15. A method according to claim 14 for controlling weeds in crops of cereals and rice.

16. Use of a phenoxyphenoxyalkanoylthioamide according to claim 1 for controlling weeds in crops of cereals and rice.

**Revendications**

1. Nouveaux phénoxy-phénoxy-alcanoyl-thioamides de formule I

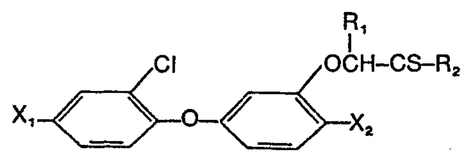

(I)

dans laquelle

$X_1$ représente le chlore ou un groupe trifluorométhyle,

$X_2$ représente le chlore ou un groupe nitro,

$R_1$ représente un groupe alkyle en C1–C4,

$R_2$ représente le groupe amino ou un groupe amidino

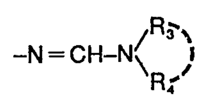

dans lequel

$R_3$ et $R_4$ représentent chacun, individuellement, l'hydrogène ou un groupe alkyle en C1–C4, ou forment ensemble un groupe alkylène en C4–C5.

2. Amides selon la revendication 1, de formule Ia

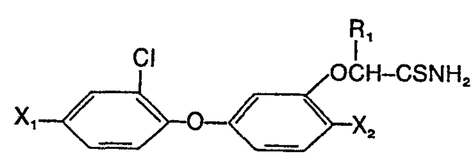

(Ia)

dans laquelle $X_1$, $X_2$ et $R_1$ ont les significations indiquées dans la revendication 1.

3. Amidines selon la revendication 1, de formule Ib

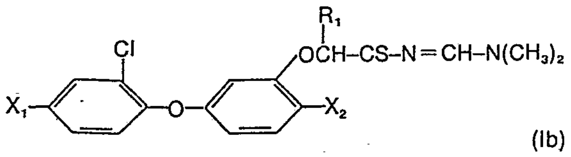

(Ib)

dans laquelle $X_1$, $X_2$ et $R_1$ ont les significations indiquées dans la revendication 1.

4. L'α-[3-(2'-chloro-4'-trifluorométhyl-phénoxy)-6-chlorophénoxy]-thiopropionyl-amide selon la revendication 1.

5. L'α-[3-(2',4'-dichlorophénoxy)-6-chloro-phénoxy]-thiopropionyl-amide selon la revendication 1.

6. L'α-[3-(2',4'-dichlorophénoxy)-6-nitro-phénoxy]-thiopropionyl-amide selon la revendication 1.

7. L'α-[3-(2',4'-dichlorophénoxy)-6-chloro-phénoxy]-thiobutyryl-amide selon la revendication 1.

8. L'α-[3-(2'-chloro-4'-trifluorométhylphénoxy)-6-chlorophénoxy]-thiopropionyl-diméthylamidine selon la revendication 1.

9. L'α-[3-(2',4'-dichlorophénoxy)-6-chloro-phénoxy]-thiopropionyl-diméthylamidine selon la revendication 1.

10. L'α-[3-(2',4'-dichlorophénoxy)-6-nitro-phénoxy]-thiopropionyl-diméthylamidine selon la revendication 1.

11. L'α-[3-(2',4'-dichlorophénoxy)-6-chloro-phénoxy]-thiobutyryl-diméthylamidine selon la revendication 1.

12. Procédé de préparation des nouveaux phénoxy-phénoxy-alcanoyl-thioamides de formule I, revendication 1, caractérisé en ce que l'on condense un 3-(2-chlorophénoxy)-phénol de formule II

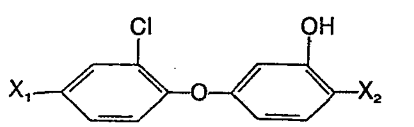

(II)

dans laquelle $X_1$ et $X_2$ ont les significations indiquées dans la revendication 1, avec un nitrile d'acide α-halogéno-(alcane en C1–C4)-carboxylique de formule III,

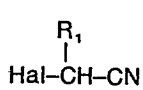

(III)

dans laquelle Hal représente un atome d'halogène et $R_1$ a les significations indiquées dans la revendication 1, ce qui donne un α-[3-(2-chlorophénoxy)-phénoxy-alcanoylnitrile de formule IV

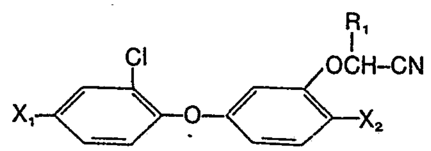

(IV)

dans laquelle $X_1$, $X_2$ et $R_1$ ont les significations indiquées dans la revendication 1, qu'on traite dans un solvant basique inerte par le sulfure d'hydrogène jusqu'à conversion du nitrile en thioamide, après quoi, le cas échéant, on fait encore réagir l'amide obtenu de formule Ia

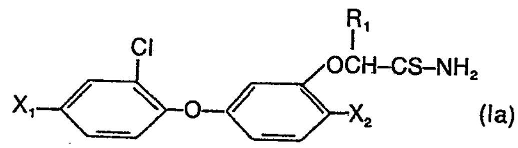

(Ia)

dans laquelle $X_1$, $X_2$ et $R_1$ ont les significations indiquées dans la revendication 1, avec un formamide de formule V

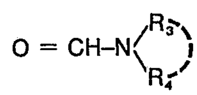

(V)

dans laquelle $R_3$ et $R_4$ ont les significations indiquées dans la revendication 1, ou avec un dérivé réactif d'un tel formamide, en présence d'un solvant organique inerte et d'un agent de condensation basique.

13. Produit herbicide caractérisé en ce qu'il contient en tant que composant actif, avec des véhicules et diluants inertes, un phénoxy-phénoxy-alcanoyl-thioamide de formule I.

14. Procédé pour la lutte sélective contre les mauvaises herbes dans la culture de végétaux utiles, caractérisé en ce que l'on traite les cultures ou l'aire de culture par une quantité efficace d'un phénoxy-phénoxy-alcanoyl-thioamide selon la revendication 1.

15. Procédé selon la revendication 14, pour la lutte contre les mauvaises herbes dans les cultures de céréales et de riz.

16. Utilisation des phénoxy-phénoxy-alcoxy-thioamides selon la revendication 1 pour la lutte contre les mauvaises herbes dans les cultures de céréales et de riz.